# EUROPEAN PATENT APPLICATION

(11) **EP 1 604 695 A1**
(43) Date of publication of application: **14.12.2005**
(21) Application number: 04711695.9
(22) Date of filing: 17.02.2004
(51) Int. Cl.: A61L 27/38, A61L 15/00

(54) **AMNION-ORIGIN MEDICAL MATERIAL AND METHOD OF PREPARING THE SAME**

(30) Priority: 26.02.2003 JP 2003049947
(71) Applicant: Amniotec Inc., Tokyo 102-0073 (JP); Kinoshita, Shigeru, Osaka-shi Osaka 5450035 (JP)
(72) Inventor: KINOSHITA, Shigeru, Kyoto-shi, Kyoto 6068264 (JP); NAKAMURA, Takahiro, Kyoto 6068305 (JP)
(74) Representative: Müller Fottner Steinecke
(86) International application number: PCT/JP2004/001692
(87) International publication number: WO 2004/078225

(57) **Abstract**

It is intended to provide an amnion-origin medical material which can be easily handled and fully sterilized and, moreover, favorably acts as a base material for forming a cell layer thereon. This material is prepared by: (i) removing the epithelial layer from the amnion while remainingat at leaset a part of the base membrane thereof; and (ii) drying it under such conditions that the remaining base membrane can sustain a structure allowing the adhesion and proliferation of cells thereon in using.

## Description

### TECHNICAL FIELD

The present invention relates to a medical material derived from amniotic membrane and the use thereof. The medical material of the present invention can be used for, for example, an injury repairing material, an injury protective material, an injury curing material, an adhesion preventive material, an artificial tissue, and the like. Specifically, the medical material can be used for producing epithelium for transplantation (for example, corneal epithelium) or endothelium for transplantation (for example, corneal endothelium).

### BACKGROUND ART

Under circumstances of the development of technology for cell cultivation and progress in medical technology, various trials for reconstructing (reproducing) tissues that cannot exhibit their original functions have been carried out with various tissues targeted. For example, in one trial, epidermis of the skin or corneal epithelium is reconstructed on an appropriate substrate in vitro, and the reconstructed tissue is used as tissue for transplantation. Such a substrate used for constructing tissue for transplantation requires properties of having a high affinity (and low immunogenicity) with respect to a living body, and allowing the adhesion and proliferation of the targeted cells thereon. Hitherto, as a material having such properties, amniotic membrane has been widely used. Amniotic membrane is membrane that encloses a fetus together with amniotic fluid. It is thought that amniotic membrane is particularly preferred as a material for producing a graft because of its low immunogenicity. Amniotic membrane has a schematic structure in which an epithelial layer is formed on a stromal layer composed of collagen via a basement membrane. As mentioned above, amniotic membrane has low immunogenicity. However, in order to further reduce the immunogenicity and to construct tissue for transplantation by constructing a cell layer on the amniotic membrane, it is necessary to prevent cells derived from amniotic membrane from being mixed. Therefore, it is thought to be preferable that an epithelial layer is removed in use (see Japanese Unexamined Publication No. H5-56987).

### DISCLOSURE OF THE INVENTION

Amniotic membrane from which an epithelial layer has been removed is prepared as follows. Firstly, collected amniotic membrane is washed, unnecessary attached materials are removed, and then an epithelial layer is denuded. The thus obtained amniotic membrane that does not contain the epithelium is stored in refrigerating or freezing environment before use except that it is used immediately after preparation. Therefore, a satisfactory thermal management before use is required and special attention should be paid in handling (in particular, at the time of delivery). Furthermore, since the material is transplanted into a living body, a sufficient sterilization process is required to be carried out before use. According to the investigation by the present inventors, however, when amniotic membrane that does not contain the epithelium in refrigerating or freezing environment is subjected to treatment with a γ beam or an electron beam, discoloration of a solution was observed, and the amniotic membrane was not resistant to the necessary sterilization process.

With the foregoing problems in mind, the present invention was made, and it is an object of the present invention to provide a medical material derived from amniotic membrane that can be handled easily, subjected to sufficient sterilization process, and favorably acts as a substrate on which a cell layer is constructed.

The present inventors have paid attention to a basement membrane of amniotic membrane and thought it important that the basement membrane is allowed to remain and that the structure thereof is maintained in order to achieve a preferable medical material suitable as a substrate for constructing a cell layer. Furthermore, from the viewpoint of handling, the present inventors thought it most preferable that the medical material is in a dried state before use. Based on the above-mentioned thoughts, the present inventors attempted to construct a medical material capable of achieving the above-mentioned object by using amniotic membrane as a starting material. Firstly, the epithelium is removed from the amniotic membrane with at least a part of the basement membrane left. Subsequently, water component is removed by vacuum lyophilization so as to obtain amniotic membrane in a dried state (amniotic membrane that does not contain the epithelium). In order to examine the properties of the resultant amniotic membrane that does not contain the epithelium, the present inventors attempted to construct a cell layer by using the amniotic membrane as a substrate. As a result, in a model system using corneal epithelial cells, favorable adhesion of cells to the amniotic membrane and proliferation, and further stratification were observed. When transplantation experiment was carried out by using the finally obtained cell layer (corneal epithelium-like sheet) as a graft, reliable survival of the graft to a living body and excellent effect of reconstructing the cornea was confirmed. The present invention was completed based on the above-mentioned findings and provides the following configurations.
[1] A medical material derived from amniotic membrane, comprising the following properties (1) to (3) of:
   (1) being in a dried state;
   (2) not having an epithelial layer; and
   (3) having a basement membrane that retains a structure allowing the adhesion and proliferation of cells thereon in use.
[2] The medical material derived from amniotic membrane described in [1], further comprising the following property (4) that:
   (4) the medical material is contained in a container in a state that is not substantially in contact with oxygen.
[3] The medical material derived from amniotic membrane described in [1] or [2], wherein the state that is not substantially in contact with oxygen is a state in which the container has been evacuated or a state in which the gas in container has been replaced by nitrogen gas.
[4] The medical material derived from amniotic membrane described in any of [1] to [3], wherein the cell is a corneal epithelial cell, a corneal endothelial cell, or an oral mucosal epithelial cell.
[5] A method of preparing a medical material derived from amniotic membrane, the method comprising the steps (i) and (ii):
   (i) removing an epithelial layer from amniotic membrane with at least a part of a basement membrane left; and
   (ii) drying the amniotic membrane that does not contain the epithelium obtained in the step (i) under such conditions that the remaining basement membrane retains a structure allowing the adhesion and proliferation of cells thereon in use.
[6] The method of preparing a medical material derived from amniotic membrane described in [5], wherein the step (ii) is carried out by lyophilization.
[7] The method of preparing a medical material derived from amniotic membrane described in [5] or [6], further comprising the following step (iii):
   (iii) containing the dried material obtained in the step (ii) in a container in a state that is not substantially in contact with oxygen.
[8] The method of preparing a medical material derived from amniotic membrane described in [7], wherein the state that is not substantially in contact with oxygen is a state in which the container has been evacuated or a state in which the gas in container has been replaced by nitrogen gas.
[9] A method of constructing a corneal epithelium-like sheet, the method comprising the following step:
   cultivating corneal epithelial cells on the medical material described in any of [1] to [4].
[10] A method of constructing a corneal epithelium-like sheet, the method comprising the following steps (a) and (b):
   a) cultivating corneal epithelial cells or cells having differentiation potency into corneal epithelial cell-like cells on the medical material described in any of [1] to [4]; and
   b) after cells were proliferated and stratified, bringing an outermost layer of the stratified cells into contact with air.
[11] The method of constructing a corneal epithelium-like sheet described in [10], wherein the step (a) is carried out in the coexistence of supporter cells.
[12] The method of constructing a corneal epithelium-like sheet described in [10], wherein the step (a) is carried out in the coexistence of supporter cells and in a state in which an isolation membrane having a pore size through which the supporter cells cannot pass is provided between the supporter cells and the medical material.
[13] The method of constructing a corneal epithelium-like sheet according to any of [10] to [12], wherein the cell cultivated in the step (a) is an oral mucosal epithelial cell.
[14] A method of constructing a corneal endothelium-like sheet, the method comprising the following steps (A) to (C):
   (A) cultivating and proliferating collected corneal endothelial cells;
   (B) preparing a cell suspension by collecting the proliferated corneal endothelial cells; and
   (C) plating the cell suspension on the medical material described in any of [1] to [4], and cultivating thereof.
[15] The method of constructing a corneal endothelium-like sheet described in [14], wherein the following step (B-1) is carried out after the step (B):
   (B-1) increasing a cell density of the cell suspension by centrifugation.
[16] The method of constructing a corneal endothelium-like sheet described in [14] or [15], wherein, in the step (C), centrifugation is carried out after the cell suspension is plated.
[17] The method of constructing a corneal endothelium-like sheet described in [14] or [15], wherein the step (C) comprise the following steps (C-1) to (C-5):
   (C-1) placing a container in a culture container, the container having a bottom surface including a membrane with a pore size allowing a culture solution to pass through;
   (C-2) placing the medical material described in any of [1] to [4] on the bottom surface of the container;
   (C-3) plating the cell suspension on the medical material;
   (C-4) carrying out centrifugation; and
   (C-5) cultivating.

Unless otherwise specified, in this specification, "corneal epithelial cell" is used as a term encompassing cells included in a corneal epithelial cell layer. That is to say, it also includes a corneal epithelial stem cell. Similarly, "corneal endothelial cell" is used as a term encompassing cells included in a corneal endothelial cell layer. That is to say, it also includes corneal endothelium stem cell. Furthermore, in this specification, "corneal epithelium-like sheet" is a term for meaning a cell layer that has similar features to those of the corneal epithelium and can be transplanted as a substitute for the corneal epithelium. Similarly, a "corneal epithelium transplantation sheet" is used as a term for meaning a composition that has similar features to those of the corneal epithelium and can be transplanted for reconstructing the corneal epithelium. On the other hand, "corneal endothelium-like sheet" is a term for meaning a cell layer that has similar features to those of the corneal endothelium and can be transplanted as a substitute for the corneal endothelium. Similarly, a "corneal endothelium transplantation sheet" is used as a term for meaning a composition that has similar features to those of the corneal endothelium and can be transplanted for reconstructing the corneal endothelium.

### BRIEF DESCRIPTION OF THE DRAWINGS

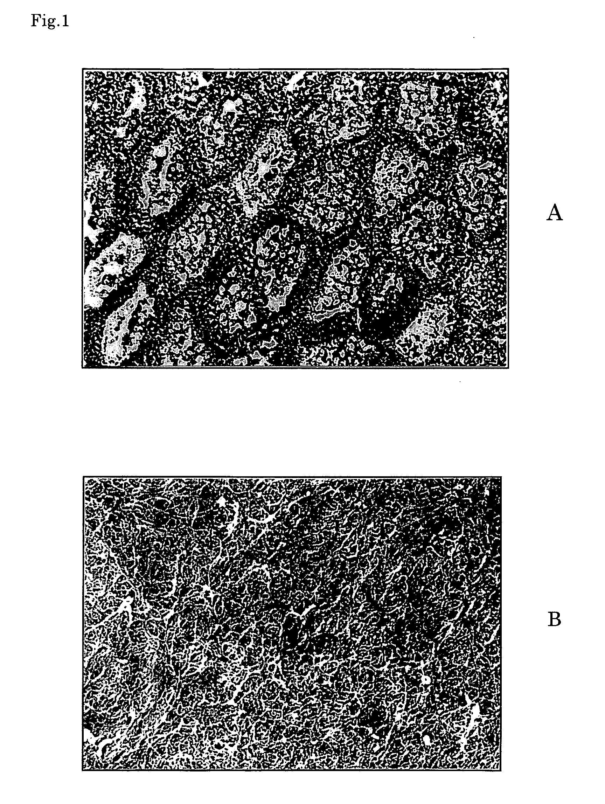

Fig. 1 shows scanning electron micrographs of images of amniotic membrane. A shows an image of the surface of amniotic membrane in a normal state (a state in which the epithelium is kept intact); and B shows an image of the surface of the amniotic membrane after the epithelium was denuded (0.02% EDTA solution). In A, polygonal amniotic membrane epithelium is observed, whereas, in B, no amniotic membrane epithelium is observed, which confirms that the epithelium was completely denuded.

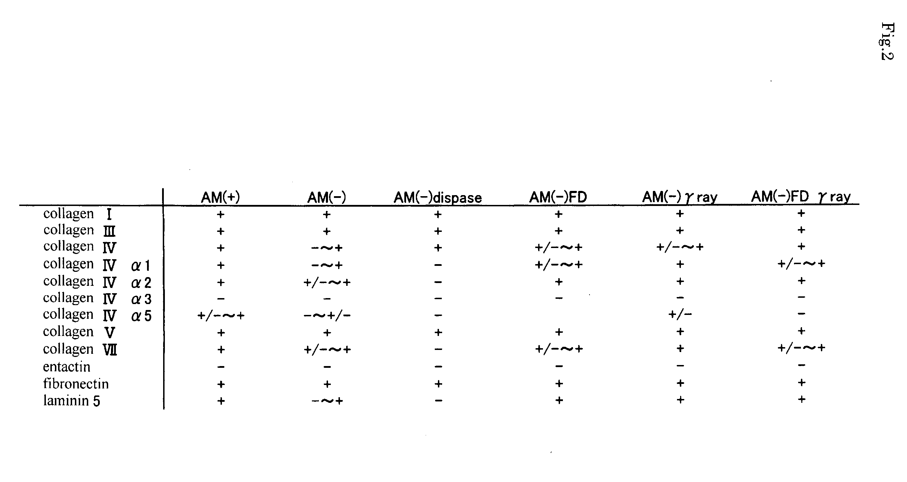

Fig. 2 is a table summarizing the surface analysis of lyophilized amniotic membrane. In the table, + denotes positive and - denotes negative. AM(-)FDyray: dried amniotic membrane obtained by the method according to Example 5, AM(+): sample of amniotic membrane subjected to only washing after collection, AM(-): sample of amniotic membrane from which the epithelium was removed but to which lyophilization was not carried out, AM(-)dispase: sample of amniotic membrane from which the epithelium was removed by dispase treatment but to which drying process is not carried out, AM(-)FD: sample of amniotic membrane from which the epithelium was removed and to which lyophilization was carried out, but sterilization process was not carried out, AM(-)γray: a sample of amniotic membrane to which sterilization process was carried out without carrying out lyophilization after the epithelium was removed.

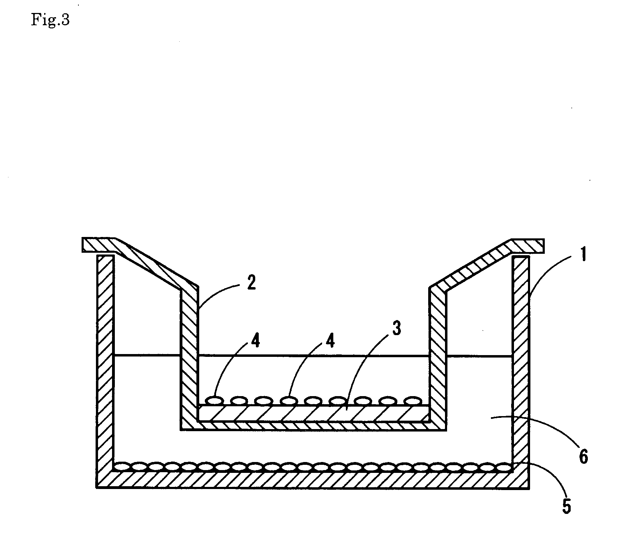

Fig. 3 is a cross-sectional view schematically showing a state of an instrument, etc. when oral mucosal epithelial cells are cultivated on amniotic membrane. In a culture dish 1, a culture insert 2 is disposed. On the bottom surface of the culture dish 1, a 3T3 cell layer 5 is formed. Furthermore, on the bottom surface of the culture insert 2, amniotic membrane 3 is placed and corneal epithelial cells 4 are cultivated on the amniotic membrane. Reference numeral 6 denotes a culture medium.

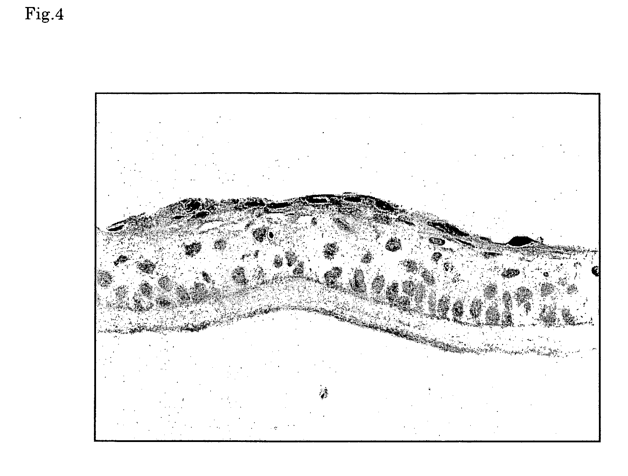

Fig. 4 shows an HE (hematoxylin-eosin) stained image of a corneal epithelial cell layer formed on lyophilized amniotic membrane.

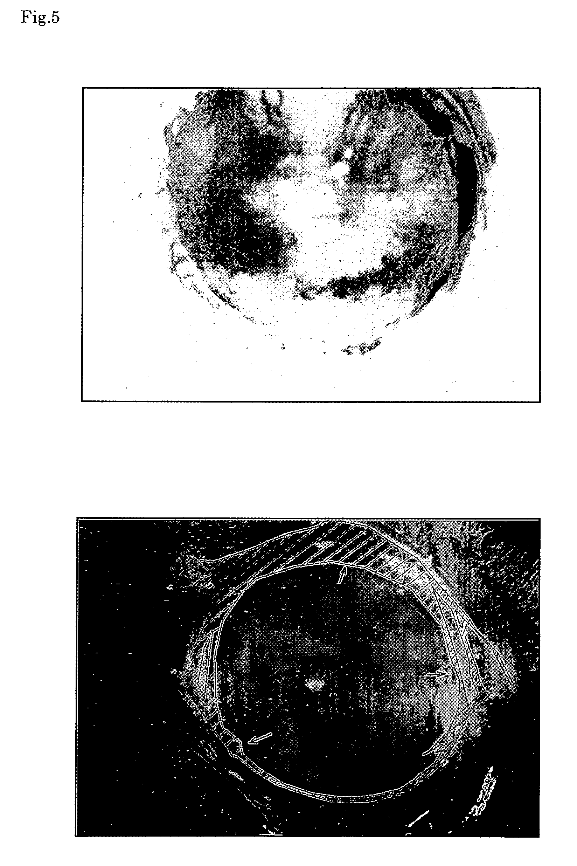

Fig. 5 shows a state of the ocular surface before operation (upper image), and a state of the ocular surface (fluorescein-stained image) two days after transplantation of a corneal epithelium transplantation sheet (lower image). A portion stained with fluorescein (arrow) is shown by hatching. The ocular surface (transplanted surface) is not stained with fluorescein, showing the survival of a graft onto the ocular surface. Furthermore, it is observed that the entire peripheral portion of the graft is stained with fluorescein.

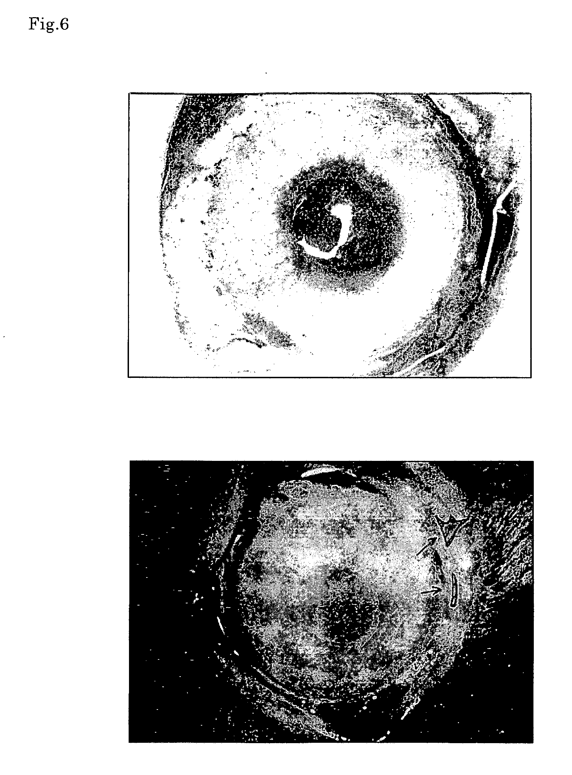

Fig. 6 shows a state of the ocular surface 7 days after transplantation of a corneal epithelium transplantation sheet (upper view shows an image obtained by directly observing the ocular surface, and lower view shows an image stained with fluorescein). It is shown that the graft maintains transparency (upper view). A portion stained with fluorescein (arrow) is shown by hatching. It is shown that only a small portion is stained and that the graft remains on the ocular surface and further spreads toward the periphery as compared with two days after the transplantation.

### BEST MODE FOR CARRYING OUT THE INVENTION

The first aspect of the present invention relates to a medical material derived from amniotic membrane including the following features (1) to (3) of:
(1) being in a dried state;
(2) not having an epithelial layer; and
(3) having a basement membrane that retains a structure allowing the adhesion and proliferation of cells thereon in use.

Firstly, the medical material of the present invention is characterized by being derived from amniotic membrane. Herein, "being derived from amniotic membrane" broadly means that the medical material is obtained by using the amniotic membrane as a starting material. From the viewpoint of immunogenicity, it is preferable that the medial material of the present invention is derived from human amniotic membrane. Human amniotic membrane is a membrane covering the outermost layer of the uterus and the placenta, and has a structure in which a basement membrane and an epithelial layer are formed on stromal tissue that is rich in collagen.

With the property (1), the medical material of the present invention can be handled easily. Specifically, the medical material can be stored even at room temperature (for example, from about 10°C to about 35°C). That is to say, the material is not required to be managed in freezing or refrigerating environment before use, thus facilitating the handling thereof (storing, delivery, etc.). However, the above-mentioned description does not mean that the medical material of the present invention cannot be stored at lower temperatures, and it may be stored in freezing or refrigerating environment if necessary.
On the other hand, since the medical material is in a dried state, it can be subjected to an effective sterilization process without affecting the use thereof. Furthermore, since the medical material is in a dried state, very little deterioration over time occurs. As a result, the medical material can be maintained at high quality for a long time.

The property (2) of the present invention contributes to lowering the immunogenicity. That is to say, amniotic membrane is a material having low immunogenicity in nature, but when amniotic membrane is deprived of an epithelial layer completely, the immunogenicity of the amniotic membrane is further reduced. On the other hand, the property of not having an epithelial layer is important in that the targeted cell layer is allowed to be favorably constructed on the medical material of the present invention. When a cell layer is intended to be constructed by plating certain cells on a substrate that is amniotic membrane with an epithelial layer left, the proliferation of the plated cells are inhibited by the epithelial cells of amniotic membrane. By using amniotic membrane from which an epithelial layer is completely removed, such a proliferation inhibition due to amniotic membrane epithelial cell may not occur. Thus, with the property of not having the epithelium, the medical material of the present invention functions as a substrate suitable for constructing a cell layer of interest.

Since the medical material of the present invention has the property (3), it becomes a material that can be preferably used as a substrate for constructing a cell layer of interest. In amniotic membrane obtained from which the epithelium has been completely removed and which is air-dried, that is, air dried amniotic membrane consisting of only stromal layer, since a basement membrane component is generally decomposed, when cells are plated thereon, the cells do not adhere favorably. However, according to the medical material of the present invention, the basement membrane is allowed to remain and the structure is allowed to be highly retained. Therefore, the cells plated on the base membrane can be adhered well by an adhesion factor contained in the basement membrane. As a result, proliferation and stratification of cells can be achieved.
Note here that the term "in use" in property (3) means the time when the medical material of the present invention is soaked in an appropriate solution (sterilized water, physiological saline solution, phosphate buffer solution, appropriate culture medium, and the like), and thereby the medical material is recovered from the dried state.

The presence of the basement membrane can be confirmed by examining the presence of constituting components peculiar to the basement membrane, e.g., type IV collagen (α1, α2 and α5), type VII collagen, laminine 5, and the like. That is to say, in the medical material of the present invention, when it is recovered, at least one, preferably a plurality of, and more preferably all of the above-mentioned components are detected. Note here that the detection of the components such as type VII collagen can be carried out by immunostaining procedure using antibodies specific to these components.

Examples of cells that are allowed to proliferate on the medical material of the present invention may include epithelial cells (corneal epithelial cells, skin epithelial cells, oral mucosal epithelial cells, and the like), and endothelial cells (corneal endothelial cells, and the like). Note here that example of the oral mucosal epithelial cells include, for example, cells existing in the dental root part (oral crevicular mucosal epithelial cells), mucosal epithelial cells of labial part, mucosal epithelial cells of palate part, mucosal epithelial cells of buccal part, and the like.
As mentioned above, medical material derived from amniotic membrane of the present invention is an extremely useful material for constructing a cell layer (tissue) that is intended to be used for transplantation.

It is preferable that the medical material of the present invention is contained in a container in a state that is not substantially in contact with oxygen. In this embodiment, the medical material is not deteriorated by oxygen and can be maintained at high quality for a long time. The term "state that is not substantially in contact with oxygen" means a state in which oxygen is not substantially present in a container. Specifically, such a state can include, for example, a state in which the container is evacuated or a state in which nitrogen is filled (nitrogen-substituted) in a container. The "container" is not particularly limited as long as it is suitable for containing the medical material of the present invention. For example, a bag or a tube made of plastic synthetic resin (a container may be formed by superimposing two sheets and sealing the peripheral portions thereof), or a bottle made of inorganic material such as glass may be used as a container of the present invention.
The above-mentioned medical material of the present invention can be produced by the following method (a second aspect of the present invention).

The second aspect of the present invention relates to a method of producing a medical material derived from amniotic membrane and the method includes the following steps (i) and (ii):
(i) removing an epithelial layer from amniotic membrane with at least a part of a basement membrane left; and
(ii) drying the amniotic membrane that does not contain the epithelium obtained in the step (i) under such conditions that the remaining basement membrane retains a structure allowing the adhesion and proliferation of cells thereon in use.
In the step (i), an epithelial layer is removed. At this time, however, basement membrane is not removed together and at least a part of the basement membrane is allowed to remain. Such a process is carried out by denuding an epithelial layer with the use of a cell scraper, forceps, etc. Preferably, the adhesion between cells constituting the epithelia layer is loosen by using EDTA, proteolytic enzyme, and the like, in advance, and then the epithelial layer is denuded with the use of a cell scraper, etc. However, it is preferable that such a pre-process (process using EDTA, etc.) is carried out under conditions where the structure of the basement membrane involved in the adhesion of epithelial layer with respect to a stromal layer is not destroyed. For example, if process is carried out using dispase under the general conditions (for example, dispase is added so that the concentration becomes 1.2 IU and reaction is carried out at 37°C for one hour), not only an epithelial layer but also basement membrane are considerably damaged. When such a pre-processing is carried out, basement membrane holding the original function cannot be allowed to remain. An important point of the step (i) in the present invention is in that an epithelial layer is completely removed while a basement membrane is allowed to remain in a state in which at least a part of the basement membrane is allowed to remain with the original function thereof maintained. For example, when the pre-process is carried out with the use of EDTA (for example, EDTA is added so that the concentration becomes 0.02% to 0.1% and reaction is carried out at 4°C to 37°C for one hour to four hours), the effect on the basement membrane is extremely small.

Preferably, the amniotic membrane used in the step (i) is human amniotic membrane. Human amniotic membrane can be collected by, for example, human embryonic membrane, placenta, etc. obtained at the time of afterbirth at delivery. Specifically, the human amniotic membrane can be prepared by treating and purifying human embryonic membrane, placenta, and umbilical cord obtained right after delivery as a whole. The method of preparing human amniotic membrane can employ a well-known method such as a method described in Japanese Patent Unexamined Publication No. H05-56987, etc. That is to say, amniotic membrane can be prepared by detaching amniotic membrane from the embryonic membrane obtained at delivery and removing the remaining tissue by a physical treatment such as ultrasonic cleansing and an enzyme treatment, and the like. The thus prepared human amniotic membrane can be cryopreserved immediately before the step (i) is carried out. The human amniotic membrane can be frozen in a liquid obtained by mixing equal volume ratio of DMEM (Dulbecco's modified Eagle's medium) and glycerol at, for example, -80°C. By the cryopreservation, not only the improvement in operation but also reduction of the antigenicity can be expected.

In the step (ii) following the step (i), the amniotic membrane that does not contain the epithelium obtained in the step (i) is dried under such conditions that the remaining basement membrane retains a structure allowing the adhesion and proliferation of cells thereon in use. The drying method is not particularly limited as long as the method can be carried out under such conditions. However, lyophilization is preferably employed. In the lyophilization, in general, in a low atmospheric pressure environment (vacuum) in which a boiling point is about -20°C (107 Pa, 0.8 Torr) to about -50°C (4 Pa, 0.03 Torr), water content is removed by sublimation from a frozen and solidified sample (for example, a sample frozen at about -40°C). According to the lyophilization, water can be removed also from the inside uniformly and high dryness can be achieved. Therefore, the material can be dried while highly maintaining the original function and form. Furthermore, the lyophilization has features: (1) less deterioration occurs during process; (2) sterilization can be achieved easily; (3) a dried material excellent in recovering property can be obtained; (4) a dried material excellent in preserving property can be obtained, and the like.

The lyophilization can be carried out by a lyophilization apparatus including a vacuum chamber, a cooling and heating device and an exhaust system (a cold trap and a vacuum pump). A number of lyophilization apparatuses are commercially available. In the step (ii) of the present invention, a lyophilization apparatus, which was arbitrarily selected from the above, can be used. Note here that the process conditions can be set based on the instruction for use attached to the apparatus to be used. At this time, size of samples subjected to drying process and dryness, etc. may be considered. The dryness may be set so that, for example, the water activity (AW) becomes less than 0.5.

It is preferable that after the above-mentioned step (ii), the following step (iii) is carried out.
(iii) containing the dried material obtained in the step (ii) in a container so that the material is not substantially in contact with oxygen.
According to this step (iii), it is possible to obtain dried amniotic membrane that does not contain the epithelium that is packaged in a state that is substantially isolated from oxygen. Thus, a medical material derived from amniotic membrane capable of being stored for a long time can be obtained.
The step (iii) can be carried out, for example, by evacuating a container or by substituting nitrogen inside the container after the dried material is contained in an appropriate container. Alternatively, it can be also carried out by enclosing deoxidant in a container. As the container, for example, a bag or a tube made of plastic synthetic resin (a container may be formed by superimposing two sheets and sealing the peripheral portions thereof), or a bottle made of inorganic material such as glass may be used as a container of the present invention.

Another aspect of the present invention relates to a method of using the medical material derived from amniotic membrane provided in the first aspect of the present invention. One embodiment is a method of constructing a corneal epithelium-like sheet. In this embodiment, on the medical material derived from amniotic membrane, corneal epithelial cells or cells having differentiation potency into corneal epithelial cell-like cells (hereinafter referred to as "cells for corneal epithelium" collectively) are cultivated. The corneal epithelial cells can be obtained from an appropriate donor's cornea. It is preferable to use a recipient's own corneal epithelial cells if possible. It is advantageous because a corneal epithelium-like sheet that is free from immunological rejection in transplantation can be constructed, and thus transplantation that does not involve immunological rejection can be carried out. When it is impossible or difficult to obtain a recipient's own corneal epithelial cells, corneal epithelial cells of a person other than a recipient may be used. However, in this case, it is preferable to select a donor by considering immunological compatibility.

The "cells having differentiation potency into corneal epithelial cell-like cells" mean cells capable of constructing a corneal epithelium-like cell layer in the cultivation in vitro or in the body after transplantation. Herein, the "corneal epithelium-like cell layer" is a cell layer provided with various properties (for example, cells have high transparency, cells of the uppermost layer are not cornified, barrier function is provided, and the like) necessary for functioning as the corneal epithelium.
The present inventors have confirmed that oral mucosal epithelial cells have differentiation potency into such corneal epithelial cell-like cells (see PCT/JP02/11857). It is suggested that oral mucosal epithelium has stem cells and it is thought that oral mucosal epithelium easily induce differentiation into cells forming an epithelium-like cell layer. Furthermore, the use of oral mucosal epithelial cells has the following advantages: they can be collected easily; a large number of cells can be collected; and when a patient with bilateral ocular disease is treated, a transplantation material can be prepared from cells derived from the autologous cells. In particular, with the advantage that a transplantation material derived from autologous cells can be used in a patient from which corneal epithelial cells cannot be collected, it is expected that the clinically extremely important problem about rejection reaction can be significantly solved.

As the oral mucosal epithelial cells, cells existing in the dental root part (oral crevicular mucosal epithelial cells), cells of labial part, cells of palate part, cells of buccal part, and the like, can be used. Among them, it is particularly preferable to use oral crevicular mucosal epithelial cells because of the high proliferation ability and low antigenicity. The oral mucosal epithelial cells can be collected by ablating a site where targeted cells exist with the use of a scalpel, or by scraping it out. Oral crevicular mucosal epithelial cells can be collected by separating oral mucosal epithelial cells from the enamel cement transition portion and collecting the cells from the obtained tissue piece. Note here that in order to remove impurities such as connective tissue, preferably, a treatment with enzyme such as dispase or trypsin, etc., filtration treatment are carried out.
It may be possible to use oral mucosal epithelial cells collected from the oral cavity of a person other than a patient to whom the corneal epithelium-like sheet constructed in the present invention is to be transplanted. However, taking immunological rejection into consideration, it is preferable that oral mucosal epithelial cells are collected from the patient's own oral cavity and cultivated.

Cells for corneal epithelium are cultivated on the medical material derived from amniotic membrane of the present invention. That is to say, the collected cells for corneal epithelium are plated on the medical material derived from amniotic membrane and cultivated under appropriate cultivation conditions. The cells for corneal epithelium can be plated on the medical material derived from amniotic membrane so that the cell density becomes, for example, about 1×10³ cells/cm² or more, preferably in the range from about 1×10³ cells/cm² to about 1×10⁷ cells/cm², and further preferably in the range from about 1×10⁴ cells/cm² to about 1×10⁶ cells/cm².

It is preferable that the cells for corneal epithelium are cultivated in the presence of supporter cells. The supporter cell is referred to as also a feeder cell and supplies a culture medium with growth factor, etc. When the cells for corneal epithelium are cultivated in the coexistence of supporter cells, it can be expected that the proliferation efficiency of the oral mucosal epithelial cells is improved. As the supporter cells, for example, a 3T3 cell (Swiss mouse 3T3 cell, mouse NIH3T3 cell, 3T3J2 cell, etc.) and the like, may be used. Among them, it is preferable to use a mouse NIH3T3 cell as a supporter cell from the viewpoint of proliferation efficiency, ease in handling, etc.

It is preferable that the supporter cells have been inactivated by using mitomycin C, etc. It is advantageous because the inhibition of the proliferation of the cells for corneal epithelium due to the proliferation of the supporter cells themselves is prevented, and the proliferation efficiency of the cells for corneal epithelium is enhanced. Such inactivation can be also carried out by a radiation treatment, etc.
When the cells for corneal epithelium are cultivated in the coexistence of supporter cells, it is preferable that an isolation membrane having a pore size through which the supporter cells cannot pass is provided between the supporter cells and the collagen layer. The use of the isolation membrane makes it possible to prevent the supporter cells from entering the side of a medical material derived from amniotic membrane (i.e. the side of cells for corneal epithelium) at the time of cultivation. As a result, the supporter cells may not be mixed in the finally obtained corneal epithelium-like sheet. This means that a corneal epithelium-like sheet being free from problem of immunological rejection by supporter cells can be constructed, which is clinically extremely significant.

As the isolation membrane, an isolation membrane having a pore size through which the supporter cells cannot pass can be used by selecting the known membrane appropriately. For example, a polycarbonate membrane having a pore size of about 0.4 µm to 3.0 µm can be used. A material of the isolation membrane is not particularly limited. Besides polycarbonate, polyester and the like may be used. Such the isolation membranes are on the market and easily available.
An example of the culture method using an isolation membrane may include the following method. Firstly, inactivated supporter cells are plated and cultivated on a container such as a dish (a first container), thereby forming a layer of supporter cells on the surface of the container. Then, a second container, which has a bottom surface made of an isolation membrane, is set in the first container so that the bottom surface of the second container is located in a culture medium. Subsequently, on the bottom surface of the second container, that is, on the isolation membrane, a medical material derived from amniotic membrane is placed. Then, on the medical material derived from amniotic membrane, cells for corneal epithelium are plated and cultivated.
On bottom surface of the second container, a medical material derived from amniotic membrane is previously placed (drying process may be carried out after the medical material derived from amniotic membrane is previously placed). This second container is set in the first container in which supporter cells are plated, and then on the medical material derived from amniotic membrane, cells for corneal epithelium may be plated and cultivated.

The cell density of the supporter cells may be, for example, about 1×10² cells/cm² or more, preferably in the range from about 1×10² cells/cm² to about 1×10⁷ cells/cm², and further preferably in the range from about 1×10³ cells/cm² to about 1×10⁵ cells/cm². As to the ratio with respect to the number of the cells for corneal epithelium, cultivation may be carried out under conditions where the number of the supporter cells to be used may be, for example, 1/10³ times to 1×10² times, and preferably 1/10² times to 1 time as the number of the cells for corneal epithelium. When the number of the supporter cells is small, the proliferation rate of the cells for corneal epithelium is lowered, and when it is too small, excellent stratification of the cells for corneal epithelium cannot be obtained. On the other hand, it is not preferable that the number of the supporter cells is too large, because the proliferation rate of the cells for corneal epithelium is lowered.

The culture medium used for cultivating the cells for corneal epithelium is not particularly limited as long as the cells can proliferate and be stratified. For example, a medium, in which DMEM (Dulbecco's modified Eagle's medium) that is generally used for growing epithelial cells and Ham's F12 media are mixed with each other at the predetermined ratio, and FBS, growth factor, antibiotics and the like are added, may be used. Specific examples include a mixed medium of DMEM and Ham's F12 medium (mixing volume ratio of 1 : 1) containing FBS (10%), insulin (5 mg/ml), cholera toxin (0.1 nM), epithelial cell growth factor (EGF) (10 ng/ml) and penicillin-streptomycin (50 IU/ml). Furthermore, a mixed medium of DMEM and Ham's F12 media further containing triiodothyronine (e.g. 2 nM), glutamine (e.g. 4 mM), transferrin (e.g. 5 mg/ml), adenine (e.g. 0.18 mM), and/or hydrocortisone (e.g. 0.4 mg/ml) may be used.

By cultivating the cells for corneal epithelium on the medical material derived from amniotic membrane (in step (a)), cells for corneal epithelium proliferate and are stratified on the medical material derived from amniotic membrane. Then, a step (step (b)) of bringing the surface layer of the stratified cells into contact with the air is carried out in order to induce the differentiation of cells forming a cell layer and barrier function. Not here that this step herein is also referred to as air lifting.
This step (b) can be carried out by lowering the surface of the culture medium by temporarily removing a part of the culture medium by using a dropper, a pipette, and the like, thereby temporarily exposing the outermost layer of the cell layer to the outside of the culture medium. Alternatively, this step can be carried out by lifting up the cell layer together with the medical material derived from amniotic membrane, the outermost layer is allowed to temporarily expose from the surface of the culture medium. Furthermore, by using the tube etc., the air may be fed into the culture medium so as to bring the uppermost layer of the cell layer into contact with the air. From the viewpoint of the ease in operation, it is preferable that a method of lowering the surface of the culture medium, thereby exposing the outermost layer of the cell layer to the outside is carried out.
The period when this step (b) is carried out, that is, the period of time when the uppermost layer of the stratified cell layer is brought into contact with the air differs depending upon the state of the cells or culture conditions, etc. but it may be for example 3 days to 3 weeks, preferably 5 days to 2 weeks, and further preferably for about one week.

According to the above-mentioned method of the present invention, on the medical material derived from amniotic membrane, a cell layer (corneal epithelium-like sheet), in which cells derived from cells for the corneal epithelium are stratified, is formed. This corneal epithelium-like sheet together with the medical material derived from amniotic membrane that are used as a substrate can be used as a transplantation material (substitute for the corneal epithelium) for patients with injured or defective cornea, etc. In this case, the corneal epithelium-like sheet is transplanted to the corneal epithelium defective part so that a layer of the medical material derived from amniotic membrane is located to the side of the eyeball. In transplantation, it is preferable to promote survival of the graft by fixing it to the surrounding tissue with a surgical suture. Furthermore, it is preferable that after transplantation, the surface of the transplanted part is protected by temporarily being covered with a therapeutic contact lens.
Note here that a graft from which a part of the medical material derived from amniotic membrane has been removed or all of the medical material derived from amniotic membrane has been removed (that is, only a cell layer) may be used. The medical material derived from amniotic membrane can be removed by appropriately combining a chemical treatment with EDTA, etc., an enzymatic treatment by proteolytic enzyme, etc., and a physical treatment such as denuding by using forceps.

According to the above-mentioned method, a corneal epithelium-like cell layer (corneal epithelium-like sheet) in which cells derived from cells for corneal epithelium are stratified, is constructed. However, it is thought that the method of the present invention can provide a cornea transplantation material (a corneal epithelium transplantation sheet), in which the cell layers are formed on the medical material derived from amniotic membrane. That is to say, the method of the present invention can be used as a method of constructing the corneal epithelium transplantation sheet having such a structure.

When oral mucosal epithelial cells are used for the cells for corneal epithelium, in a corneal epithelium-like sheet, cells derived from the oral mucosal epithelial cells are stratified. Whether or not the cells are derived from the oral mucosal epithelial cells can be confirmed by using, as an index, the expression of keratin 4 or keratin 13 specific to the oral mucosal epithelial cells in the cells constituting the corneal epithelium-like sheet. Alternatively, the expression of keratin 3 can be used as an index. This keratin 3 is known to be one of the cornea-specific keratins. However, keratin 3 was confirmed to be expressed in the oral mucosal epithelial cell. Note here that it is preferable that the oral mucosal epithelial cell is used as a material for producing a composition for transplantation of corneal epithelium because it expresses this cornea-specific keratin, keratin 3.
On the other hand, by examining the expression of genes specific to the oral mucosal epithelial cells, it can be confirmed that cells forming a cell layer are derived from the oral mucosal epithelial cells.

Preferably, the corneal epithelium-like sheet of the present invention includes one or more than one of the following properties or characteristics. Particularly preferably, the corneal epithelium-like sheet of the present invention has all of the following characters or properties.
(1) The cells of the uppermost layer are not cornified. This is one of the features of corneal epithelium. When this feature is observed, the corneal epithelium-like sheet of the present invention is similar to the corneal epithelium and is expected to exhibit the same function as that of the corneal epithelium. Note here that "cornified" is also referred to as "keratinized", which represents the phenomenon in which keratin is generated in a cell and the cell organelle such as nucleus is lost. It can be confirmed whether or not the cells are cornified by confirming, for example, flatness of cell or the presence of nucleus in a cell.
(2) The cells of the uppermost layer are flat-shaped. That is to say, a cell layer derived from cells for forming cornea is configured by forming a layer of cells having flat shape on a layer of cells having approximately cuboidal shape. It is thought that when the uppermost layer is covered with flat-shaped cells, the tightness between cells is increased and a below-mentioned barrier function is attained. Also in the corneal epithelium, cells in the uppermost layer are flat-shaped. When this feature is observed, the corneal epithelium-like sheet of the present invention is similar to corneal epithelium and is expected to exhibit the same function as that of corneal epithelium.
(3) A barrier function is provided. The barrier function means a function of preventing liquid, gas or the like from infiltrating from the surface layer or a function of preventing liquid from releasing via the surface layer. When such barrier function is provided, it is possible to maintain moisture (tear) on the surface after transplantation and to prevent more than necessary moisture from being released. Cornea can maintain moisture on the surface thereof as it has a barrier function, and thereby it resists blinking. Therefore, providing the barrier function is one of the most important features required for a corneal epithelium transplantation material. When this feature is observed, the corneal epithelium-like sheet of the present invention is similar to the corneal epithelium and is expected to exhibit the same function as that of the corneal epithelium. Whether or not this barrier function is provided can be examined based on the extent of infiltration of solution including a labeling material such as Horseradish peroxidase.

Another embodiment of the medical material derived from amniotic membrane of the present invention relates to a method of constructing a corneal endothelium-like sheet. In this embodiment, by cultivating corneal endothelial cells on the medical material derived from amniotic membrane, a corneal endothelium-like cell layer is constructed. Specifically, the corneal endothelium-like sheet can be constructed by the following procedures.

### <1> Collection and proliferation of corneal endothelial cell

Corneal endothelial cells are collected in the usual manner from the cornea of a recipient him/herself or an appreciate donor. For example, Descemet membrane and the endothelial cell layer of a corneal tissue are peeled off from the corneal stroma, then transferred to a culture dish and treated with dispase, etc. Thus, corneal endothelial cells are peeled off from the Descemet membrane. The corneal endothelial cells residing in the Descemet membrane can be peeled off by pipetting and the like. The Descemet membrane is removed, thereafter corneal endothelial cells are cultivated in an appropriate culture solution in which the endothelial cells can be grown. As the culture solution, it is possible to use, for example, a commercially available DMEM (Dulbecco's Modified Eagle's Medium) containing FBS (fetal bovine serum), b-FGF (basic-fibloblast growth factor), EGF (epidermal growth factor), insulin and antibiotics such as penicillin and streptomycin, etc. It is preferable to use a culture container (culture dish) the, surface of which is coated with type I collagen, type IV collagen, fibronectin, laminin or extracellular matrix of bovine endothelial cells, etc. It is advantageous because the adhesion of the corneal endothelial cells to the surface of the culture container is promoted, thus enabling excellent proliferation.

The temperature conditions for cultivating corneal endothelial cells are not particularly limited as long as the corneal endothelial cell can be grown. The temperature is, for example, about 25°C to about 45°C. When considering the proliferation efficiency, the temperature is preferably about 30°C to about 40°C and more preferably about 37°C. Cultivation time is different depending upon, for example, states of cells to be used, but it is, for example, 7 to 14 days.

Herein, it is preferable to use recipient's own corneal endothelial cells if possible. It is advantageous because a corneal endothelium-like sheet, which may be free from immunological rejection when it is employed for transplantation, can be constructed, and thereby transplantation that does not cause immunological rejection can be performed. When it is impossible or difficult to obtain corneal recipient's own endothelial cells, corneal endothelial cells of a person other than a recipient may be used. However, in this case, it is preferable to select a donor by considering immunological compatibility.

### <2> Subculture and production of cell suspension

After the corneal endothelial cells used for culture are proliferated, subculture of the cells can be carried out. Subculture is carried out when cells become subconfluent or confluent. The subculture can be carried out as follows. Firstly, cells are peeled off from the surface of the culture container by treating with trypsin-EDTA etc. Then, cells are collected. A culture solution is added to the collected cells to make a cell suspension. It is preferable that centrifugation is carried out when or after cells are collected. With such a centrifugation, cell suspension with a high cell density can be prepared. Note here that conditions for centrifugation can include 500 rpm (30g) to 1000 rpm (70g) and 1 to 10 minutes.
As in the above-mentioned initial culture, the cell suspension is plated on the culture container and cultivated. Subculture can be carried out in the same culture conditions as in the above-mentioned initial culture. Cultivation time is different depending upon, for example, states of cells to be used, but it is, for example, 7 to 21 days. The above-mentioned subculture can be carried out plural times if necessary. By repeating subcultures, the number of cells can be increased and a cell suspension with a high cell density can be prepared. Finally, it is preferable to prepare a cell suspension with a cell density of about 5×10⁵ cells/ml to 20×10⁵ cells/ml.

### <3> Plating and cultivation of cell suspension

A cell suspension is plated on a medical material derived from amniotic membrane and cultivated. At this time, the number of cells to be plated is adjusted so that a cell layer with a desired cell density can be formed in the finally constructed corneal endothelium-like sheet. Specifically, for example, about 3000 cells/mm² to about 7500 cells/mm², preferably about 5000 cells/mm² to about 7500 cells/mm² are plated so that a cell layer with a cell density of about 2000 cells/mm² to about 4000 cells/mm² is formed. The cultivation is carried out under the same conditions as in the above-mentioned initial culture, and the like. Cultivation time is different depending upon, for example, states of cells to be used, but it is, for example, 3 to 21 days.

In the medical material derived from amniotic membrane, it is preferable that corneal endothelial cells are plated at the side of a surface from which the epithelium has been removed and exposed to the surface (i.e., the side of basement membrane). It is advantageous because it is thought that the side of this surface contains a large amount of type IV collagens and adhesion and proliferation of the plated corneal endothelial cells can be carried out well.

Cultivation of the corneal endothelial cells on the medical material derived from amniotic membrane can be carried out, for example, by the following procedures. Firstly, a container (hereinafter, also referred to as "culture insert") having a bottom surface including membrane having a pore size allowing a culture solution to pass is placed in a culture container with the bottom surface faced downward. Then, medical material derived from amniotic membrane is disposed on the bottom surface of the culture insert (inner side of the culture insert). Then, on the medical material derived from amniotic membrane, a cell suspension is plated and cultivated. Note here that the medical material derived from amniotic membrane may be formed on the bottom surface of the culture insert in advance. That is to say, for example, a cell suspension may be plated and cultivated after a medical material derived from amniotic membrane is disposed on the bottom surface of the culture insert (drying process may be carried after the material is placed), and then this culture insert is set in a culture container.
An example of membrane that can be used for the bottom surface of the culture insert can include a polycarbonate membrane having a pore size of about 0.4 µm to 3.0 µm. Besides, a polyester membrane may be used. Such membranes are on the market and easily available.

Centrifugation may be carried out after the cell suspension is plated in the culture insert. Thereby, the cell density on the medical material derived from amniotic membrane can be increased. Furthermore, adhesion of cells to the surface of the medical material derived from amniotic membrane becomes excellent. Furthermore, it is possible to obtain an effect of preventing the cell density from being concentrated on one part. Note here that conditions of centrifugation herein can include, for example, 500 rpm (30b) to 1,000 rpm (70g) and 1 to 10 minutes.

By carrying out the cultivation as mentioned above, a corneal endothelium like sheet, in which a cell layer made of cells derived from corneal endothelium is formed on a medical material derived from amniotic membrane, can be formed. The corneal endothelium-like sheet can be used as a transplantation material (substitute for the corneal endothelium) for treating diseases that need transplantation of corneal endothelium, for example, diseases such as bullous keratopathy, corneal edema, corneal leukoma, keratoconus, and the like. Note here that a graft from which a part of the medical material derived from amniotic membrane is removed or all of the medical material derived from amniotic membrane is removed (that is, only the corneal epithelium-like sheet) may be used. The medical material derived from amniotic membrane can be removed by appropriately combining a chemical treatment with EDTA, etc., an enzymatic treatment by proteolytic enzyme, etc., and a physical treatment such as denuding by using forceps.

Cultivation (including initial culture, subculture and cultivation on the medical material derived from amniotic membrane) of corneal endothelial cells can be carried out in the coexistence of supporter cells. By cultivating the corneal endothelial cells in the coexistence of supporter cells, effects of improving the proliferation efficiency and promoting the differentiation of the corneal endothelial cells can be expected. As the supporter cells, for example, 10T1/2 fibroblast (Hyldahl L: Primary cell cultures from human embryonic corneas. J Cell Sci. 66: 343-351, 1984), a 3T3 cell (Swiss mouse 3T3 cell, mouse a NIH3T3 cell, 3T3J2 cell, etc.), a corneal stroma cell, and the like, may be used.
When the corneal endothelial cells are cultivated in the coexistence of supporter cells, it is preferable that an isolation membrane having a pore size through which the supporter cells cannot pass is provided between the corneal endothelial cells and the supporter cells. The use of this isolation membrane makes it possible to prevent the supporter cells from entering the side of the corneal endothelial cells at the time of cultivation. As a result, the supporter cells may not be mixed in the finally obtained corneal endothelium-like sheet. This means that a corneal endothelium-like sheet being free from problem of immunological rejection by the supporter cells can be constructed, which is clinically significant so much.

When supporter cells are used, it is preferable that the supporter cells are inactivated with mitomycin C, etc. It is advantageous because the inhibition of the proliferation of the corneal endothelial cells due to the proliferation of the supporter cells themselves is prevented. Such inactivation may also be carried out by a radiation treatment, etc.

As the isolation membrane, a membrane similar to the membrane used in the above-mentioned culture insert can be employed. That is to say, for example, a membrane having a pore size of about 0.4 µm to 3.0 µm made of polycarbonate, polyester, etc. can be used.

The cell density of the supporter cells may be, for example, about 1×10² cells/cm² or more, preferably in the range from about 1×10² cells/cm² to about 1×10⁷ cells/cm², and further preferably in the range from about 1×10³ cells/cm² to about 1×10⁵ cells/cm². It is not preferable that the number of the supporter cells is small, because it is thought that the proliferation rate of the corneal endothelial cells is lowered. On the other hand, it is not preferable that the number of the supporter cells is too large, because the proliferation rate of the corneal endothelial cells is rather lowered.

An example of methods for transplanting the corneal endothelium-like sheet constructed as mentioned above may include full thickness trepanation and deep keratectomy. In the former method, full thickness cornea is once collected by using a trephine and a corneal endothelial cell layer is replaced by the corneal endothelium-like sheet of the present invention, followed by returning the full thickness cornea to a recipient. Specifically, the method can be carried out as follows. Firstly, section of the full thickness cornea of a recipient (host) is carried out by using a trephine, and a part (or entire) of the cornea is collected in a button shape. Then, from the collected cornea section, Descemet membrane and a corneal endothelial cell layer are peeled off. Onto the exposed corneal stroma, the corneal endothelium-like sheet of the present invention is allowed to adhere, so that the medical material derived from amniotic membrane is located at the side of corneal stroma. Thereafter, the cornea section is returned to the recipient and fixed thereto with suture.
On the other hand, in the deep keratectomy, instead of extracting the full thickness of the cornea, only the deep portion of the cornea is excised. It is thought that by this method, burden to a recipient is reduced. Specifically, the method can be carried out as follows. Firstly, a part of the cornea stroma of a recipient is peeled off, and a part of the deep cornea stroma and a part of Descemet membrane and endothelial cell layer are excised. Note here that only the endothelial cell layer or only the endothelial cell layer and Descemet membrane may be peeled off and excised. Only the injured corneal endothelial cells of the recipient may be peeled off. Next, the corneal endothelium-like sheet of the present invention is inserted into the excised portion by using a spatula, etc. If necessary, air is filled in the anterior chamber and the graft is fixed.
Note here that it can be confirmed whether or not the transplanted corneal endothelium-like sheet exhibits the barrier function and pump function similarly to the corneal endothelial cell layer in a living body by examining the change in the thickness of the cornea after transplantation and the occurrence of edema.
Hereinafter, the present invention will be described in detail with reference to the following Examples.

### <Example 1> Collection of amniotic membrane

After giving a pregnant woman who does not have a systemic complication and would undergo caesarean section sufficient informed consent together with an obstetrician in advance, amniotic membrane was obtained at the time of the caesarean section in the operation room. The operation was carried out cleanly. In accordance with the operation work, the operators washed hands, and then wore a special gown. Before delivery, a clean vat for obtaining amniotic membrane and physiologic saline for washing were prepared. After delivery, the placenta tissue was transferred to the vat and amniotic membrane tissue was manually detached from the placenta. A portion where amniotic membrane and placenta were strongly attached to each other was separated from each other with scissors.

### <Example 2> Treatment of amniotic membrane

Treatment process of amniotic membrane included: (1) washing, (2) trimming, and (3) storing sequentially in this order. Throughout all the processes, operation is desired to be carried out in a clean draft. For all containers and instruments for use, sterilized ones were used, and for dishes, etc. sterilized disposable ones were used. The obtained amniotic membrane was washed for removing blood component attached thereto and further washed with a sufficient amount of physiological saline (0.005% ofloxacin was added). Then, the amniotic membrane was washed three times in total with a phosphate buffer solution (PBS) containing penicillin-streptomycin (50 IU). Then, the amniotic membrane was transferred into a dish and cut and divided into the size of about 4×3 cm with scissors. After the amniotic membranes were divided, amniotic membranes in good conditions were selected based on the shape, thickness, or the like.

### <Example 3> Storage of amniotic membrane

One cc each of stock solution was placed in 2 cc sterilized cryotube and one sheet each of amniotic membrane, which had been obtained, washed and selected, was placed and labeled, and then stored in a deep freezer at -80°C. For the stock solution, 50% sterilized glycerol in DMEM (Dulbecco's Modified Eagle Medium: GIBCOBRL) was used. The expiration date for use of stored amniotic membrane was determined at 3 months and expired amniotic membrane was disposed of by incineration. Note here that instead of such storing treatment, the following treatment to the epithelium may be carried out.

### <Example 4> Treatment of amniotic epithelium

Amniotic membrane stored at -80°C was thawed at room temperature, and then washed twice with a phosphate buffer solution (PBS) containing penicillin-streptomycin (50 IU). Amniotic membrane after washing was soaked in a 0.02% EDTA solution (Nacalai tesque) (in 100 mm culture dish) and reacted in a CO₂ incubator at 37°C for one hour. After reaction, the amniotic membrane was washed twice with a sufficient amount of PBS, and then the epithelium was manually denuded out by using a cell scraper (Nunc, USA). Note here that, it was confirmed that one layer of the amniotic epithelium was completely denuded by this procedure process by optical microscope and electron microscope (scanning electron microscope) operations (see Fig. 1). Note here that Fig. 1 shows scanning electron micrographs of images of amniotic membrane. A shows an image of the surface of amniotic membrane in a normal state (a state in which a treatment of the epithelium was not carried out); and B shows an image of the surface of the amniotic membrane after the epithelium was denuded (0.02% EDTA solution).

### <Example 5> Preparation of lyophilized amniotic membrane

Amniotic membrane from which the epithelium had been removed was sandwiched by a pair of sterilized plastic frames, and then fixed with clip. Each frame of amniotic membrane was transferred into -80°C deep freezer. After it was confirmed that the amniotic membrane was frozen, lyophilization was carried out (-110°C for one hour) by using a vacuum lyophilization machine (Yamato, NEOCOOL). In accordance with the instruction, the conditions were set so that sufficient dried substances were obtained. The amniotic membrane after lyophilization was taken out from the frame and transferred to a two-layered bag consisting of an outer layer of polyamidonylon and an inner layer of polyethylene and vacuum-packed by using a household vacuum pack (MAGIC VAC, manufactured by FLAEM MOUVA). The thus obtained vacuum-packed amniotic membrane was irradiated with γ ray (about 25 kGy) so as to be sterilized. The amniotic membrane after sterilization was stored at room temperature in a vacuum-packed state immediately before use. Note here that even after 12 months of storing, the state right after lyophilization was maintained. Later experiment was carried out by using the dried amniotic membrane that had been stored at room temperature for one month.

### <Example 6> Surface analysis of lyophilized amniotic membrane

In the dried amniotic membrane obtained in Example 5, in order to confirm that basement membrane remained on the surface at the side from which the epithelium had been removed and the structure thereof was maintained, immunological analysis was carried out as follows. Firstly, the dried amniotic membrane was soaked in PBS and recovered, followed by cutting it in appropriate size so as to be freeze-embedded in an OCT compound. Thereafter, the embedded amniotic membrane was sliced with a cryostat so as to form slide sections. The slide sections were washed with PBS, followed by blocking with 1% fetal bovine serum (FBS) so as to suppress unspecific antibody reaction. Thereafter, primary antibody was reacted at room temperature for one hour. As the primary antibodies, antibodies (COSMO BIO CO., LTD.) that specifically bind to type I to V and VII collagens, entactin, and laminine 5, respectively, were used. After reaction, the reactant was washed with PBS containing triton-X three times for 15 minutes, and a fluorescent labeled antibody (secondary antibody) was reacted at room temperature for one hour. Then, the reactant was washed with PBS three times for 15 minutes and enclosed, and then the tissue was observed by a con-focal microscope. As a control group, sample (AM(+)) of amniotic membrane subjected to only washing after collection, sample (AM(-)) of amniotic membrane from which the epithelium was removed but to which lyophilization was not carried out, sample (AM(-)dispase) of amniotic membrane from which the epithelium was removed by dispase treatment but to which drying process is not carried out, sample (AM(-)FD) of amniotic membrane from which the epithelium was removed and to which lyophilization was carried out, but sterilization process was not carried out, sample (AM(-)γray) of amniotic membrane to which sterilization process was carried out without carrying out lyophilization after the epithelium was removed were prepare. Each sample was subjected to a primary antibody reaction and a secondary antibody reaction by the same method as mentioned above. Fig. 2 shows a table summarizing the results of immunological analysis. In the table, + denotes positive and - denotes negative. Note here that the dried amniotic membrane obtained by the method according to Example 5 is represented by AM(-)FDγray.
As shown in this table, in a sample (AM(-)) from which the epithelium had been removed, type IV collagen (α2 and α5), type V collagen, type VII collagen and laminine 5, which are components peculiar to the basement membrane, were detected. Furthermore, these components were detected similarly in the sample (AM(-)FD) of amniotic membrane that was subjected to lyophilization after the epithelium had been removed and the sample (AM(-)FDγray) of amniotic membrane that was further subjected to sterilization process. These results shows that by employing the treatment for removing epithelium according to Example 4, the epithelium was removed with basement membrane left. Furthermore, it is found that even when lyophilization after the epithelium was removed, a state in which the basement membrane remains can be maintained, and even when further sterilization process is carried out, remaining basement membrane is hardly damaged. On the other hand, in the sample (AM(-)dispase) of amniotic membrane from which the epithelium had been removed by using dispase, components peculiar to the basement membrane were not detected. This is thought to show that the basement membrane was destroyed by the dispase treatment.
From the above-mentioned analysis results, it was confirmed that the dried amniotic membrane prepared in the above-mentioned method had a basement membrane having an original structure.

### <Example 7> Collection of corneal epithelial cells

The cornea collected from a rabbit (6-week age, Japanese white color species) was soaked in DMEM containing 10% fetal bovine serum (FBS) and unnecessary tissues such as conjunctiva, corneal endothelium, etc. were excised. Thereafter, the tissues were washed with phosphate buffer solution (PBS) and soaked in phosphate buffer solution (PBS) containing 1.2 U/ml dispase (Nacalai tesque) at 37°C for one hour. The tissues after treatment were taken out and soaked in 0.02% EDTA at room temperature for two minutes and then in phosphate buffer solution at room temperature for two minutes so as to stop the dispase activity. In DMEM containing 10% fetal bovine serum (FBS), corneal epithelial cells were denuded, followed by centrifugation so as to condense and collect the corneal epithelial cells.

### <Example 8> Preparation of co-cultivated cells

As co-cultivated cells (supporter cells), NIH-3T3 cells (hereinafter abbreviated as "3T3 cells") were used. The 3T3 cells that had been cultivated previously and became confluent were soaked in a 0.05% mitomycin C solution in 75F flask (BD Falcon) for two hours so as to suppress the proliferation activity of 3T3. Then, the cells were washed several times with a phosphate buffer solution (PBS) so as to remove mitomycin C. The cells were treated with a 0.05% trypsin-EDTA solution, followed by pipetting so as to obtain a cell suspension (3T3 cell suspension).

### <Example 9> Construction of corneal epithelium-like sheet

The lyophilized amniotic membrane obtained in Example 5 was soaked in PBS at room temperature until it is fully recovered. By using the thus prepared amniotic membrane as a substrate, corneal epithelial cell and 3T3 cells were co-cultivated by the following procedures. For a culture instrument, a 6-well culture dish (Corning, NY) and a culture insert (culture insert container) (made of polycarbonate, average pore size: 3.0 µm; manufactured by Corning, NY) were used.
Firstly, 3T3 cell suspension was plated on a culture dish so that the cell density became about 1×10⁴ cells/cm² and cultivated at 37°C under 5% CO₂. Furthermore, amniotic membrane was attached onto the culture insert with the basement membrane side (side in which the epithelium had existed) upwardly and dried at room temperature for 10 minutes. Thereafter, a corneal epithelial cell suspension was plated on the culture insert to which amniotic membrane was attached so that the cell density became about 1×10⁵ cell/cm².

After the above-mentioned operation, as shown in Fig. 3, the culture insert was disposed in the culture dish and 3T3 cells and corneal epithelial cells were cultivated in the same medium. Note here that Fig. 3 is a cross-sectional view schematically showing a state during cultivation. It shows a state in which a culture insert 2 is disposed in a culture dish 1, and a 3T3 cell layer 5 is formed on the bottom surface of the culture dish 1. Furthermore, it shows a state in which on the bottom surface of the culture inset 2, amniotic membrane 3 is disposed and the corneal epithelial cells are cultivated thereon. Reference numeral 6 denotes a culture medium.
As the medium, a DMEM/Ham F12 mixed medium (mixed volume ratio of 1:1) containing 10% FBS, insulin (5 mg/ml), cholera toxin (0.1 nM), penicillin streptomycin (50 IU/ml), human recombinant epithelial cell growth factor (EGF) (10 ng/ml) was used.

The cells were cultivated in the above-mentioned medium for three weeks (Submerge). Thereafter, in order to induce the differentiation of the mucosal epithelium, the cells were cultivated for one week by a so-called air-lifting method. The air-lifting method is a method of lifting the liquid surface of the culture medium to the surface of the corneal epithelial cell layer formed on amniotic membrane to thus allow the surface of the cell layer to be exposed to the air. During submerging, the culture medium was replaced with new one every other day and after carrying out the air-lifting method, the culture medium was replaced with new one every day.

### <Example 10> Verification of histological properties of corneal epithelium-like sheet

As a result of cultivation by the above-mentioned method, about 20 days after cultivation had started, a cell layer similar to the corneal epithelium was formed (see Fig. 4). It is shown that in the cell layer, similar to a normal corneal layer, 4 to 5 layers are stratified. At the side of the amniotic membrane of this cell layer, a group of relatively cuboidal-shaped cells similar to the basal cells existed. Furthermore, it was shown that the cells of the outermost layer had a flat shape but included a nucleus and that the surface thereof was not cornified unlike the skin. As mentioned above, it was confirmed that the cell layer (the corneal epithelial-like layer) was formed on the amniotic membrane.
By using a transplantation sheet, in which a corneal epithelium-like cell layer was formed on amniotic membrane, formed by the above-mentioned method (hereinafter, also referred to as "corneal epithelium transplantation sheet"), the following transplantation experiment was carried out.

### <Example 11> Transplantation experiment

Firstly, to white rabbit from which the corneal epithelial cells had been collected, all the cornea epithelium and conjunctival epithelium having a thickness of 100 µm were removed from 4-mm outside form the limbus by using a crescent knife. By this operation, since the epithelial cells including corneal epithelium stem cells are lost, it is thought that artificial exhaustion of the ocular surface stem cells was reproduced. Note here that the ocular surface before transplantation was observed (see upper image of Fig. 5).
Then, a corneal epithelium transplantation sheet was transplanted into a region somewhat inner from the limbus. In transplantation, by using 10-0 nylon fiber was used to suture the sheet and the peripheral tissue. After transplantation, on the graft, a therapeutic contact lens was placed. After the operation, antibiotics and steroid ophthalmic ointment were applied twice a day. After transplantation, the ocular surface had a transparency that was the same level as that of the corneal epithelium transplantation sheet before transplantation.

Two days after and seven days after the operation, the ocular surface which had received the transplantation was observed. Two days after the transplantation, it was confirmed that the transplanted corneal epithelium transplantation sheet maintained transparency. Furthermore, it was confirmed by fluoresceine staining that the corneal epithelium transplantation sheet remained on the ocular surface without any defect (see Fig. 5, lower image). Furthermore, since the graft was not stained with fluoresceine, it was confirmed that the corneal epithelium transplantation sheet had a barrier function similar to the corneal epithelium. Furthermore, since it was found that by the fluoresceine staining, the entire periphery of the graft was stained with fluoresceine, therefore it was confirmed that the tissue existing in the transplanted part was not contamination of the remaining conjunctival epithelium. Note here that since cells of the corneal epithelium are tightly adhered to each other, the fluoresceine pigment is not infiltrated from the surface, so that staining with fluoresceine is not observed. On the other hand, when the adhesion between cells becomes loosen or the barrier function is damaged by exfoliation of the cell itself, infiltration of the fluoresceine pigments occur, and the tissues are stained. Therefore, by examining whether or not being stained with fluoresceine, it can be confirmed whether or not the transplanted corneal epithelium transplantation sheet had a barrier function similar to the corneal epithelium.

Seven days after the transplantation, the transplanted corneal epithelium transplantation sheet was confirmed to remain on the ocular surface. Furthermore, it was observed that the corneal epithelium transplantation sheet spreads toward the periphery more than the state observed two days after the transplantation and the corneal epithelium transplantation sheet covers the entire ocular surface (see Fig. 6, lower image). It was confirmed that, as shown in Fig. 6, the transplant was not stained with fluoresceine and that a barrier function necessary to the corneal epithelium was maintained. Furthermore, the transparency was also maintained (see Fig. 6, upper image).

From the results, as mentioned above, it was confirmed that the corneal epithelium transplantation sheet constructed by using a dried amniotic membrane survived and spread on the ocular surface, and maintained transparency for a long time after the operation. That is to say, it was confirmed that the corneal epithelium transplantation sheet constructed by the above-mentioned method included a cell layer functioning well as a substitute for corneal epithelium, and was suitably used as a transplantation material for reconstructing the ocular surface when the cornea was injured and damaged.

### <Example 12> Construction of corneal epithelium-like sheet using oral mucosal epithelial cell

The oral mucosal epithelium attached to tooth pulled out from a donor is separated from the enamel cement transition portion. Note here that it is preferable that a series of operations are carried out by using sterilized instruments as antiseptically as possible.
The collected oral mucosal epithelium is soaked twice in a phosphate buffer solution (PBS) containing 50 IU/ml penicillin streptomycin and Gentacin at room temperature for 30 minutes. Thereafter, the tissue is soaked in a phosphate buffer solution (PBS) containing 1.2U Dispase (Nacalai tesque) at 37°C for one hour, and soaked and treated in 0.05% trypsin-EDTA solution (GBCOBRL) for 30 minutes so as to separate cells. An enzyme activity is stopped by soaking in DMEM containing 10% fetal bovine serum (FBS). Thereafter, excess tissues are removed by using a 60 µm cell filter so as to isolate oral mucosal epithelial cells (oral crevicular mucosal epithelial cells). By using the thus obtained oral mucosal epithelial cells, a corneal epithelium like-cell layer is formed by the same method as by the method of Examples 8 and 9.

### <Example 13> Construction of corneal endothelium-like sheet

### (13-1) Collection and initial culture of human corneal endothelial cells

Descemet membrane and endothelial cell layer are peeled out from the stromaof donor cornea tissue with a forceps and disposed in 35 mm-dish, followed by treating thereof with 1 ml (final concentration: 1.2 U/ml) of dispase that has been diluted two times with PBS without containing calcium at 37°C under 5% CO₂ for 60 minutes. With this treatment, most of corneal endothelial cells are dropped off from the Descemet membrane. In order to drop off corneal endothelial cells remaining on the Descemet membrane, pipetting is carried out. Then, the Descemet membrane is removed and corneal endothelial cells are transferred to 15 cc centrifugal tube. After a culture solution is added so that the total liquid amount is 5 ml, centrifugation is carried out at 1000 rpm, 70g, for 3 minutes. As the culture solution, DMEM (GIBCOBRL) containing 10% FBS (Dainippon Pharmaceutical Co., Ltd), 2 ng/ml b-FGF (Invitrogen), penicillin (50 U/ml)-streptomycin (50 µg/ml) (Nacalai tesque) can be used. Supernatant is removed and then culture solution is added to form about 1 ml of cell suspension. This cell suspension is plated on a 24-well culture dish coated with type IV collagen and cultivated under conditions at 37°C under 5%CO₂. Thereafter, culture solutions are exchanged with new one every 48 hours.

### (13-2) Subculture of endothelial cells

When the cultivated cells become confluent, the culture solution is removed and the cells are washed three times with 1 ml of PBS without containing calcium. After washing, 200 µl of 0.05% trypsin-EDTA is added and stood at 37°C under 5%CO₂ for 3 minutes. With this treatment, endothelial cells attached to the bottom surface of the culture dish are allowed to float. 5 ml of culture solution is added; then the cell suspension is transferred to a 15 cc centrifugation tube and centrifuged at 1000 rpm, 70 g, for 3 minutes. Supernatant is removed and then culture solution is added to form about 2 ml of cell suspension. This cell suspension is plated on a 35 mm culture dish coated with type IV collagen and cultivated at 37°C under 5%CO₂. Thereafter, a culture solution is exchanged with new one every 48 hours. When the cells become confluent, the same treatment as mentioned above is carried out, and subculture is repeated until the necessary number of cells can be obtained.

(13-3) The subcultured cells are treated with 0.05% trypsin-EDTA and then the number of cells is counted. Subsequently, supernatant is removed by centrifugation. Then, a culture solution is added so that the cell density becomes 2.0 × 10⁶ cells/ml. By using the obtained cell suspension, a corneal endothelial cell layer is produced as the following procedures. As a culture instrument, a 6-well culture dish (Corning, NY) and a culture insert (culture inserting container; made of polycarbonate; average pore size: 3.0 µm; manufactured by Corning, NY) can be used. For a culture solution, the same culture solution used as mentioned above can be used.

Firstly, a culture insert is disposed in a culture dish. Then, dried amniotic membrane, which was obtained in Example 5 and recovered in PBS, is spread and placed in the culture insert (on the bottom surface) with the side where the epithelium was present facing upward. Then, cell suspension is plated on the amniotic membrane so that the cell density becomes about 5000 cells/mm². Thereafter, the culture dish is centrifuged at 1000 rpm, 70g, for 3 minutes. After centrifugation, cultivation is carried out at 37°C under 5%CO₂. Thus, a single layer of cells is constructed.

The present invention is not limited to the description of the above embodiments. A variety of modifications, which are within the scopes of the claims and which can be easily achieved by a person skilled in the art, are included in the present invention.

### INDUSTRIAL APPLICABILITY

The present invention provides a medical material derived from amniotic membrane that can be handled easily and is fully resistant to sterilization process. In the medical material of the present invention, since the epithelium has been removed, immunogenicity is extremely low. Furthermore, since basement membrane remains at least partially in a state in which original structure is highly maintained, it favorably functions as a substrate for constructing a cell layer.

## Claims

1. A medical material derived from amniotic membrane, comprising the following properties (1) to (3) of:
(1) being in a dried state;
(2) not having an epithelial layer; and
(3) having a basement membrane that retains a structure allowing the adhesion and proliferation of cells thereon in use.

2. The medical material derived from amniotic membrane according to claim 1, further comprising the following property (4) that:
(4) the medical material is contained in a container in a state that is not substantially in contact with oxygen.

3. The medical material derived from amniotic membrane according to claim 1 or 2, wherein the state that is not substantially in contact with oxygen is a state in which the container has been evacuated or a state in which the gas in container has been replaced by nitrogen gas.

4. The medical material derived from amniotic membrane according to any of claims 1 to 3, wherein the cell is a corneal epithelial cell, a corneal endothelial cell, or an oral mucosal epithelial cell.

5. A method of preparing a medical material derived from amniotic membrane, the method comprising the steps (i) and (ii):
(i) removing an epithelial layer from amniotic membrane with at least a part of a basement membrane left; and
(ii) drying the amniotic membrane that does not contain the epithelium obtained in the step (i) under such conditions that the remaining basement membrane retains a structure allowing the adhesion and proliferation of cells thereon in use.

6. The method of preparing a medical material derived from amniotic membrane according to claim 5, wherein the step (ii) is carried out by lyophilization.

7. The method of preparing a medical material derived from amniotic membrane according to claim 5 or 6, further comprising the following step (iii):
(iii) containing the dried material obtained in the step (ii) in a container in a state that is not substantially in contact with oxygen.

8. The method of preparing a medical material derived from amniotic membrane according to claim 7, wherein the state that is not substantially in contact with oxygen is a state in which the container has been evacuated or a state in which the gas in container has been replaced by nitrogen gas.

9. A method of constructing a corneal epithelium-like sheet, the method comprising the following step:
cultivating corneal epithelial cells on the medical material described in any of claims 1 to 4.

10. A method of constructing a corneal epithelium-like sheet, the method comprising the following steps (a) and (b):
a) cultivating corneal epithelial cells or cells having differentiation potency into corneal epithelial cell-like cells on the medical material described in any of claims 1 to 4; and
b) after cells were proliferated and stratified, bringing an outermost layer of the stratified cells into contact with air.

11. The method of constructing a corneal epithelium-like sheet according to claim 10, wherein the step (a) is carried out in the coexistence of supporter cells.

12. The method of constructing a corneal epithelium-like sheet according to claim 10, wherein the step (a) is carried out in the coexistence of supporter cells and in a state in which an isolation membrane having a pore size through which the supporter cells cannot pass is provided between the supporter cells and the medical material.

13. The method of constructing a corneal epithelium-like sheet according to any of claims 10 to 12, wherein the cell cultivated in the step (a) is an oral mucosal epithelial cell.

14. A method of constructing a corneal endothelium-like sheet, the method comprising the following steps (A) to (C):
(A) cultivating and proliferating collected corneal endothelial cells;
(B) preparing a cell suspension by collecting the proliferated corneal endothelial cells; and
(C) plating the cell suspension on the medical material described in any of claims 1 to 4, and cultivating thereof.

15. The method of constructing a corneal endothelium-like sheet according to claim 14, wherein the following step (B-1) is carried out after the step (B):
(B-1) increasing a cell density of the cell suspension by centrifugation.

16. The method of constructing a corneal endothelium-like sheet according to claim 14 or 15, wherein, in the step (C), centrifugation is carried out after the cell suspension is plated.

17. The method of constructing a corneal endothelium-like sheet according to claim 14 or 15, wherein the step (C) comprise the following steps (C-1) to (C-5):
(C-1) placing a container in a culture container, the container having a bottom surface including a membrane with a pore size allowing a culture solution to pass through;
(C-2) placing the medical material described in any of claims 1 to 4 on the bottom surface of the container;
(C-3) plating the cell suspension on the medical material;
(C-4) carrying out centrifugation; and
(C-5) cultivating.
